# EUROPEAN PATENT SPECIFICATION

(11) **EP 1 235 543 B1**
(45) Date of publication and mention of the grant of the patent: **10.11.2004**
(21) Application number: 00982549.8
(22) Date of filing: 08.12.2000
(51) Int. Cl.: A61K 6/00

(54) **CALCULUS CONTROL DENTURE ADHESIVE COMPOSITIONS**
ZAHNPROTHESENHAFTMITTEL ZUR KONTROLLE DER ZAHNSTEINBILDUNG
COMPOSITIONS DE CIMENT ANTI-TARTRE POUR PROTHESE DENTAIRE

(30) Priority: 08.12.1999 US 169702 P
(43) Date of publication of application: 04.09.2002
(73) Proprietor: THE PROCTER & GAMBLE COMPANY, Cincinnati, Ohio 45202 (US)
(72) Inventor: RAJAIAH, Jayanth, N., Loveland, OH 45140 (US); GILDAY-WEBER, Kimberly, Ann, Cincinnati, OH 45204 (US); ERNST, Lisa, Catron, Cincinnati, OH 45243 (US); WHITE, Donald, James, Jr., Fairfield, OH 45014 (US); GLANDORF, William, Michael, Mason, OH 45040 (US)
(74) Representative: Clemo, Nicholas Graham
(86) International application number: PCT/US2000/033414
(87) International publication number: WO 2001/041712

(56) References cited:
- US-A- 4 138 477
- US-A- 4 929 690
- US-A- 5 753 723

## Description

### BACKGROUND OF THE INVENTION

Ordinary removable dentures, dental plates and the like, comprise teeth mounted in a suitable plate or base. Denture stabilizers are used to fill the interstices between the dentures and the gums or tissues. Prior to placement of the denture in the oral cavity, a denture stabilizer is applied to the denture-plate surface which, for a perfect fit, should uniformly contact the gums and mucous tissues. The denture stabilizer is formulated not only for its adherent properties, but also to provide a cushion or gasket between the denture and the gums or tissues, thereby positioning the denture securely in the oral cavity.

Considerable effort has been made over the years to develop improved denture adhesive compositions. Both synthetic and natural polymers and gums have been used alone, in combination, and in combination with various adhesives and other materials in an attempt to improve hold and reduce oozing of the adhesive from under the dental plate, messiness and difficulty of removing the residual adhesive from the mouth and dentures. For example, alkyl vinyl ether-maleic copolymers and salts thereof are known for providing good hold in denture adhesive compositions. Such disclosures include: U.S. Patent 3,003,988, Germann et al., issued October 10, 1961; U.S. Patent 4,980,391, Kumar et al., issued December 25, 1990; U.S. Patent 5,073,604, Holeva et al., issued December 17, 1991; U.S. Patent 5,525,652, Clarke, issued June 11, 1996; U.S. Patent 5,340,918, Kittrell et al., issued Aug. 23, 1994; U.S. Patent 5,830,933, Synodis et al., issued Nov. 3, 1998.

In addition to adhesion, it is desirable to deliver anticalculus benefits in a denture adhesive composition especially for those denture wearers who still have some natural teeth remaining. Mature calculus consists of an inorganic portion which is largely calcium phosphate arranged in a hydroxyapatite crystal lattice structure similar to bone, enamel and dentine. An organic portion is also present and consists of desquamated epithelial cells, leukocytes, salivary sediment, food debris, and microorganisms.

It is generally known that certain polyphosphates are effective anticalculus agents when delivered to the oral cavity by incorporation into aqueous based compositions like dentifrice or mouth rinse compositions. For example US 5,096, 699, issued March 17, 1992, Gaffar et al, assigned to Colgate, teaches dentifrice and mouth rinse compositions comprising azacycloalkane-2,2-diphosphonic acids (AAP) and salts thereof and a synthetic anionic polymeric polycarboxylate (SAPP). In addition, US 4,913,895, Miyake et al., issued April 3, 1990, teaches dentifrice and mouth rinse compositions with selected polyphosphates and menthol, anethol, or mixtures. These references, however, only disclose the delivery of polyphosphates via aqueous dentifrice and mouth rinse compositions, where the delivery is also dependent, at least in part, on brushing or rinsing of the oral cavity. The prior art, then, does not suggest that anticalculus agents would be available and delivered in sufficient quantities to render an anticalculus benefit, when placed in a non-aqueous composition, also comprising adhesive components.

Despite the above-noted technologies, as well as others, a need still exists for denture stabilizing compositions providing both superior hold and anticalculus benefits to the denture wearer. In accordance with the present invention, denture adhesive compositions comprising a denture adhesive component combined with an anticalculus agent provide anticalculus benefits, while providing superior denture hold.

### SUMMARY OF THE INVENTION

The present invention relates to a non-aqueous denture adhesive composition comprising:
a) from 20 to 50%, by weight of the composition, of a denture adhesive component selected from salts of copolymers of an alkyl vinyl ether and maleic acid or maleic anhydride; salts of terpolymers of an alkyl vinyl ether, maleic acid or maleic anhydride and isobutylene; and mixtures thereof;
b) from 4.5 to 40%, by weight of the composition, of an anticalculus agent selected from salts of linear polyphosphates, having a chain length of two or more phosphate groups, and salts of diphosphonates; and
c) a non-aqueous denture adhesive carrier.

### DETAILED DESCRIPTION OF THE INVENTION

A detailed description of essential and optional components of the present invention is given below.

### Definitions

The term "anticalculus" agent, as used herein, means a material effective in reducing, controlling, inhibiting, preventing, and/or minimizing mineral (e.g., calcium phosphate) deposition related to calculus formation. The term " safe and effective adhesive amounts" as used herein means an amount sufficient to provide adherence to the oral cavity and/or provide adherence of a dental prosthesis to the oral cavity, without toxicity to the user or damage to oral tissue.

The term "AVE/MA" as used herein refers to alkyl vinyl ether-maleic acid or anhydride copolymer. The term "AVE/MA/IB'' refers to terpolymers with alkyl vinyl ether, maleic acid or anhydride, and isobutylene. The term "mixed polymer salts" or "mixed salts", as used herein, refers to salts of AVE/MA and/or salts of AVE/MA/IB where at least 2 different cations are mixed on the same polymer with each other or with other salts.

The term "free acid" or "FA" component, as used herein, refers either to the unreacted carboxyl groups (-COOH) of AVE/MA copolymer and/or AVE/MA/IB plus any other monovalent cations of carboxyl groups (e.g., COONa) of the polymer. Monovalent cations include Group IA cations, such as sodium, potassium, hydrogen, etc. Preferably, the term ''free acid" refers to the unreacted carboxyl groups (-COOH) of AVE/MA and/or AVE/MA/IB plus sodium and potassium cations. More preferably, the term "free acid" refers only to the unreacted carboxyl groups (-COOH) of the AVE/MA and/or AVE/MA/IB.

The percentages used herein to describe the cationic salt function of the alkyl vinyl ether-maleic acid or anhydride copolymers are defined as the stoichiometric percent of the total initial carboxyl groups reacted on the polymer.

All other percentages used herein are by weight of the composition unless otherwise indicated.

### Denture Adhesive Components

The present invention comprises from 20% to 50%, by weight of the composition of a denture adhesive component according to claim 1.

"Denture adhesive components" are selected from salts of AVE/MA, salts of AVE/MA/IB, and mixtures thereof.

### Alkyl Vinyl Ether-Maleic Copolymer

In one embodiment of the invention, the denture adhesive is salts of AVE/MA. The alkyl vinyl ether-maleic acid co-polymer comprises or consists essentially of the repeated structural unit: 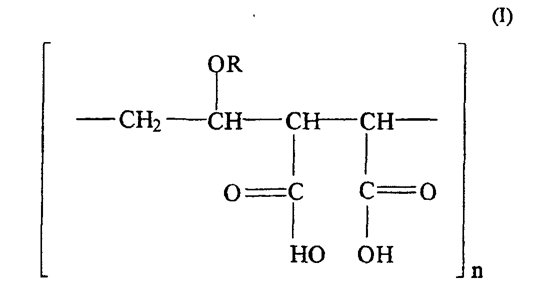 wherein R represents an alkyl radical, preferably a C₁ to C₅ alkyl radical, n is an integer greater than one representing the number of repeated occurrences of the structural unit in a molecule of the polymer.

In one embodiment, the adhesive component is mixed salts of AVE/MA, wherein the copolymer contains a cationic salt function comprising a cation selected from the group consisting of Group IA and Group 2A cations of the periodic table, yttrium, titanium, zirconium, vanadium, chromium, manganese, iron, nickel, copper, zinc, boron, aluminum, cations and mixtures thereof. In another embodiment, the adhesive component is a mixed salt of AVE/MA containing a cationic salt function comprising a cation selected from the group consisting of strontium, zinc, iron, boron, aluminum, vanadium, chromium, manganese, nickel, copper, yttrium, titanium, magnesium, calcium, sodium, cations and mixtures thereof, and in yet another embodiment the cation is selected from the group consisting of strontium, zinc, iron, magnesium, calcium, sodium, cations, and mixtures thereof.

AVE/MA contains a cationic salt function comprising from about 5% to about 50%, in another embodiment, from about 10% to about 40%, in yet another embodiment, from about 10% to about 35% (of the total initial carboxyl groups reacted) zinc cations. These zinc cations can be mixed with other cations selected from the group consisting of: from about 5% to about 65%, preferably from about 10% to about 60%, strontium cations, from about 0.001% to about 2.5%, preferably from about 0.01% to about 2% of iron, boron, aluminum, vanadium, chromium, manganese, nickel, copper, yttrium, and/or titanium cations, from about 5% to about 65%, preferably from about 15% to about 50% of calcium and/or magnesium cations.

AVE/MA and salts thereof and AVE/MA/IB and salts thereof, are also described in U.S. Patent Nos. 5,073,604 to Holeva et al., issued 12/17/91; 5,525,652, issued Jun. 11, 1996, Clarke et al.; 4,758,630, issued July 19,1988, Shah et al.; 5,304, 616, issued April 19, 1994, Rajaiah et al.; 5,424,058, issued June 13, 1995, Rajaiah; 5,424,058, issued 6/13/95, Rajaiah et al.; 4,758,630, issued July 19, 1988, Shah et al.; 5,830,933, issued Nov. 3, 1998, Synodis et al.; 2,047,398, issued July 14, 1936, Voss et al.; 3,003,988, issued Oct. 10, 1961, Germann et al.; 5,880,172. Rajaiah et al., issued March 9, 1999; 5,900,470, Prosise et al., issued 5/4/99; 5,037,924, Tazi et al., issued 8/6/91; 5,082,913, Tazi et al, issued 1/21/92; all of which are incorporated herein by reference in their entirety. Salts of AVE/MA are also described in P&G copending applications serial numbers: 06/152,158, filed Sept. 2, 1999, Rajaiah et al.; 60/129,164, filed April 14, 1999, Rajaiah et al.; 60/129,162, filed April 14, 1999, Rajaiah et al.; 60/152,122, filed Sept. 2, 1999, Rajaiah et al.; 09/291,554, filed April 14, 1999, Rajaiah et al.; 09/389,209, filed Sept. 2, 1999, Rajaiah et al.; and 09/389,210, filed Sept. 2, 1999, Rajaiah et al..

In one embodiment the free acid level of the salts of the AVE/MA or AVE/MA/IB is at least about 36%, in another embodiment is from about 36% to about 60%, and even in another embodiment is from about 40% to about 55%, of the total initial carboxyl groups of the copolymer or terpolymer.

The specific viscosity of the starting copolymer acid or copolymer anhydride is from about 1.2 to about 14, when preferably measured in a 1% weight/volume solution in MEK (methyl ethyl ketone) at 25°C. Other methods and solvents can be used to measure the specific viscosity such as a 1% weight/volume solution in DMF (dimethyl formamide) at 25°C and a 1% weight/volume solution in 2-butanone at 25°C.

Suitable AVE/MA copolymers may be prepared by well-known methods of the prior art; see, for example, US 2,782,182, and US 2,047,398.

The salt form of the subject polymers may be prepared by the interaction of the acid or anhydride polymer with at least one cationic salt function as described above, having a functional group typical of reactants of a carboxylic acid, such as, for example, the hydroxide, oxide, acetate, halide, lactate, etc. in an aqueous medium. In one embodiment, the zinc oxide, strontium carbonate, iron sulfate n-hydrate, etc. are utilized.

Ions that form toxic, irritating or contaminating by-products should be avoided, or special precautions and treatment provided to assure the removal and absence of such by-products from the polymeric salt end-product. The particular compound used should be substantially pure to assure obtaining a substantially pure, polymeric salt end-product.

The salt form of the polymer can be made by mixing the salts (sodium hydroxide, zinc oxide, strontium carbonate, ferric sulfate n-hydrate, calcium hydroxide and/or magnesium oxide, etc.) in an aqueous dispersion. This is combined with the powder alkyl vinyl ether-maleic acid or anhydride copolymer, in the form of a slurry, in an amount sufficient to provide the desired cationic content desired in the end-product. This is done at ambient temperature and then slowly heated to 70°-95°C with continuous vigorous mixing so as to prevent localized precipitation of the cationic polymeric salt; mixing is continued to ensure that all the salt forming compound is reacted with the copolymer.

Alternatively, the AVE/MA copolymer is hydrolyzed and neutralized in an aqueous mixture or slurry of one or more divalent and/or monovalent metal bases by heating the copolymer/base mixture to a temperature ranging from about 45°C to about 100°C.

In either of the above processes, the resulting slurry or solution is transferred to shallow stainless steel drying trays and placed in a forced air mechanical convection oven at 60-70°C for a time sufficient to evaporate the reaction medium (water) and remove water from the copolymer (about 18-24 hours). Alternatively, the resulting slurry or solution can be drum-dried at 100° to 200°C with hot steam to evaporate the water content and recover the copolymer in the flake form. After drying, the polymer forms brittle flakes which can easily be peeled off from the trays or drum surface and ground to a fine powder as desired to provide satisfactory denture stabilizing properties. Methods of making these mixed salts of AVE/MA polymers are further disclosed in U.S. Patent Nos. 5,073,604, Holeva et al., issued Dec. 17, 1991 and 5,872,161, Liang et al., issued Feb. 16, 1999.

### The Anticalculus Agent

The present compositions comprise a safe and effective amount of at least one anticalculus agent. This amount is from 4,5% to 40% by weight of the composition. An effective amount of the anticalculus agent is released from the denture adhesive composition. The anticalculus agent should also be essentially compatible with the other components of the composition.

The anticalculus agent is selected from salts of linear polyphosphates having a chain length of two or more phosphate groups, and salts of diphosphonates.

### Polyphosphate

In one embodiment of the present invention, the anticalculus agent is a polyphosphate. A polyphosphate within the scope of the present invention understood to consist of two or more phosphate molecules arranged in a linear configuration. Linear polyphosphates correspond to (X PO₃)ₙ where n is about 2 to about 125, wherein preferably n is greater than 4, and X is for example sodium, potassium, etc. For (X PO₃)ₙ when n is at least 3 the polyphosphates are glassy in character. Counterions for these phosphates may be the alkali metal, alkaline earth metal, ammonium, C₂-C₆ alkanolammonium and salt mixtures. Polyphosphates are generally employed as their wholly or partially neutralized water soluble alkali metal salts such as potassium, sodium, ammonium salts, and mixtures thereof. The inorganic polyphosphate salts include alkali metal (e.g. sodium) tripolyphosphate, tetrapolyphosphate, dialkyl metal (e.g. disodium) diacid, trialkyl metal (e.g. trisodium) monoacid, potassium hydrogen phosphate, sodium hydrogen phosphate, and alkali metal (e.g. sodium) hexametaphosphate, and mixtures thereof. Polyphosphates larger than tetrapolyphosphate usually occur as amorphous glassy materials. In one embodiment the polyphosphates are those manufactured by FMC Corporation which are commercially known as Sodaphos (n≈6), Hexaphos (n≈13), and Glass H (n≈21), and mixtures thereof. The polyphosphate source will comprise from 4,5 to 40% by weight of the composition.

The phosphate sources are described in more detail in Kirk & Othmer. *Encyclopedia of Chemical Technology,* Fourth Edition, Volume 18, Wiley-Interscience Publishers (1996), pages 685-707.

In one embodiment the polyphosphates are the linear "glassy" polyposphates having the formula:

XO(XPO₃)ₙX

wherein X is sodium or potassium; and n averages from about 6 to about 125.

According to claim 1, the level of anticalculus agent is from 4,5% to 40%, by weight of the composition. Polyphosphates are disclosed in US 4,913,895.

### Pyrophosphate

The pyrophosphate salts useful in the present compositions include, alkali metal pyrophosphates, di-, tri-, and mono-potassium or sodium pyrophosphates, dialkali metal pyrophosphate salts, tetraalkali metal pyrophosphate salts, and mixtures thereof. In one embodiment the pyrophosphate salt is selected from the group consisting of trisodium pyrophosphate, disodium dihydrogen pyrophosphate (Na₂H₂P₂O₇), dipotassium pyrophosphate, tetrasodium pyrophosphate (Na₄P₂O₇), tetrapotassium pyrophosphate (K₄P₂O₇), and mixtures thereof. The pyrophosphate salts are described in more detail in Kirk & Othmer, *Encyclopedia of Chemical Technology*, Third Edition, Volume 17, Wiley-Interscience Publishers (1982), pages 685-707.

In one embodiment, the compositions of the present invention comprise tetrasodium pyrophosphate. Tetrasodium pyrophosphate may be the anhydrous salt form or the decahydrate form, or any other species stable in solid form in the present compositions. The salt is in its solid particle form, which may be its crystalline and/or amorphous state, with the particle size of the salt preferably being small enough to be aesthetically acceptable and readily soluble during use.

The level of pyrophosphate salt in the compositions of the present invention is from 4,5% to 40%.

### Other Anticalculus Agents

Diphosphonate salts include azocycloalkane-2,2-diphosphonic acid salts; azocycloalkane-2,2-diphosphonic acid salts (such as those which the alkane moiety has five, six or seven carbon atoms, in which the nitrogen atom is unsubstituted or carries a lower alkyl substitutent, e.g. methyl), salts of azacyclohexane-2,2-diphosphonic acid, azacyclopentane-2,2-diphosphonic acid salts, N-methyl-azacyclopentane-2,3-diphosphonic acid salts, EHDP (ethanehydroxy-1,1-diphosphonic acid) salts, AHP (azacycloheptane-2,2-diphosphonic acid; a.k.a. 1-azocycloheptylidene-2,2-diphosphonic acid) salts, ethane-1-amino-1,1-diphosphonate salts, dichloromethane-diphosphonate salts etc.

Azacycloalkane-2,2-diphosphonic acids are disclosed in US 3,941,772, issued March 2, 1976, Ploger et al., assigned to Henkel and US 3,988,443, issued Oct. 26, 1976, Ploger et al..

### Non-Aqueous Denture Adhesive Carrier

The non-aqueous denture adhesive carrier is selected from the group consisting of a non-aqueous vehicle and a non-adhesive self supporting layer. The level of non-aqueous vehicle is from 10% to about 90%, in another embodiment is from about 20% to about 80%, and in yet another embodiment is from about 20% to about 60%, by weight of the composition.

### Non-aqueous Vehicles

The non-aqueous vehicle is generally any chemical in any physical form that does not contain water. The non-aqueous vehicle is selected from the group consisting of liquid petrolatum, petrolatum, mineral oil, glycerin, natural and synthetic oils, fats, silicone and silicone derivatives, polyvinylacetate, natural and synthetic waxes such as animal waxes like beeswax, lanolin and shellac, hydrocarbons-hydrocarbon derivatives, vegetable oil waxes such as carnauba, candelilla and bayberry wax, vegetable oils such as caprylic/capric triglycerides, in another embodiment is selected from the group consisting of liquid petrolatum, petrolatum, mineral oil, vegetable oils such as corn, soy bean, cottonseed, castor, palm and coconut oils and animal oil such as fish oil and oleic acid, and mixtures thereof; and in yet another embodiment is mineral oil.

Caprylic/capric triglycerides are triglycerides of medium chain fatty acids where the ―C==O―R group is 8-10 carbons and is obtained by the addition of glycerol to a mixture of capric and caprylic acids:
Caprylic acid: CH₃(CH₂)₆CO₂H
Capric acid: CH₃(CH₂)₈CO₂H

Therefore, vegetable oils comprised of saturated medium chain fatty acids such as caprylic acid, capric acid and mixtures thereof, can be used in the present invention. These vegetable oils and other non-aqueous vehicles for denture adhesive compositions are further described in US Patent No. 5,561,177, issued on Oct. 1, 1996, Khaledi et al., which is incorporated herein by reference in its entirety.

### Non-Adhesive Self-Supporting Layer

The non-aqueous carrier can comprise at least one non-adhesive self-supporting layer. The non-adhesive self-supporting layer is characterized by its ability to maintain strength and provide integrity for the adhesive composition in the presence of water and/or saliva. The non-adhesive self-supporting layer may include materials such as polyester, polypropylene, nylon, rayon, cellulose acetate, non-adhesive cellulose derivatives, cloth, fibrous fleece, paper, plastic, leather, microcrystalline wax, synthetic fibers, natural fibers, and mixtures thereof. Preferred are non-adhesive cellulose derivatives, polyester, polypropylene, nylon, rayon, cloth, paper, microcrystalline wax, and mixtures thereof. More preferred are polyester, polypropylene, rayon, nylon, cloth and paper.

The non-adhesive self-supporting layer may be in any physical form suitable for providing strength and/or integrity to the present adhesive compositions. Such physical forms include non-woven, woven, continuous, chopped, foam, and combinations thereof. In addition, the non-adhesive self-supporting layer may be formed by any process commonly known in the art. Such processes include un-bonded, spraybonded, spun-bonded, needle-punched, carded, thermal bonded hydroentangled, meltblown, aperture print bonded, needled, wet-laid, dry-laid, and combinations thereof.

The present denture adhesive compositions which comprise a non-adhesive self-supporting layer may also comprise a coating which is sticky to dry dentures and, if present, will be placed on one side of the denture adhesive composition. Compositions suitable for use as this type of adhesive layer include silicones, rubbers, petrolatum, natural polymers, synthetic polymers, and mixtures thereof. The adhesive layer may be present at a level of from about 0% to about 70%, and in another embodiment from about 0.5% to about 20%, by weight of the composition.

### Miscellaneous Carriers

Other suitable ingredients include colorants, preservatives (such as methyl and propyl parabens), thickeners such as silicon dioxide, and polyethylene glycol. Colorants, preservatives, thickeners may be present at levels of from about 0% to about 20%, by weight of the composition.

### Plasticizers

In addition one or more toxicologically-acceptable plasticizers may also be included in the present compositions. The term "toxicologically-acceptable", as used herein, is used to describe materials that are suitable in their toxicity profile for administration to humans and/or lower animals. Plasticizers that may be used in the present compositions include dimethyl phthalate, diethyl phthalate, dioctyl phthalate, glycerin, diethylene glycol, triethylene glycol, Igepal®, Gafac®, sorbitol, tricresyl phosphate, dimethyl sebacate, ethyl glycolate, ethylphthalyl ethyl glycolate, o- and p-toluene ethyl sulfonamide, and mixtures thereof. Plasticizers may be present at a level of from about 0% to about 70%, in another embodiment from about 1% to about 30%, by weight of the compositions.

### Flavors, Fragrance, Sensates

The compositions of the present invention may also include one or more components which provide flavor, fragrance, and/or sensate benefit (warming or cooling agents). Suitable components include natural or artificial sweetening agents, menthol, menthyl lactate, wintergreen oil, peppermint oil, spearmint oil, leaf alcohol, clove bud oil, anethole, methyl salicylate, eucalyptol, cassia, 1-8 menthyl acetate, sage, eugenol, parsley oil, oxanone, alpha-irisone, marjoram, lemon, orange, propenyl guaethol, cinnamon, vanillin, thymol, linalool, cinnamaldehyde glycerol acetal known as CGA, and mixtures thereof, as well as coolants. The coolant can be any of a wide variety of materials. Included among such materials are carboxamides, menthol, ketals, diols, and mixtures thereof. Preferred coolants in the present compositions are the paramenthan carboxyamide agents such as N-ethyl-p-menthan-3-carboxamide, known commercially as "WS-3", N,2,3-trimethyl-2-isopropylbutanamide, known as "WS-23," and mixtures thereof. Additional preferred coolants are selected from the group consisting of menthol, 3-1-menthoxypropane-1,2-diol known as TK-10 manufactured by Takasago, menthone glycerol acetal known as MGA manufactured by Haarmann and Reimer, and menthyl lactate known as Frescolat® manufactured by Haarmann and Reimer. The terms menthol and menthyl as used herein include dextro- and levorotatory isomers of these compounds and racemic mixtures thereof. TK-10 is described in U.S. Pat. No. 4,459,425, Amano et al., issued 7/10/84. WS-3 and other agents are described in U.S. Pat. No. 4,136,163, Watson, et al., issued Jan. 23, 1979; the disclosure of both are herein incorporated by reference in their entirety. These agents may be present at a level of from about 0% to about 50%, by weight of the composition.

### Other Optional Ingredients

The denture adhesive compositions may also be used as a denture adhesive and/or bioadhesive and comprise one or more therapeutic actives suitable for topical administration. Therapeutic actives may be present at a level of from about 0% to about 70%, by weight of the composition, and in one embodiment from about 1% to about 20% by weight of the compostion. Therapeutic actives include antimicrobial agents such as iodine, tricolsan, peroxides, sulfonamides, bisbiguanides, or phenolics; antibiotics such as tetracycline, neomycin, kanamycin, metronidazole, cetylpyridium chloride, or clindamycin; anti-inflammatory agents such as aspirin, acetaminophen, naproxen and its salts, ibuprofen, ketorolac, flurbiprofen, indomethacin, eugenol, or hydrocortisone; dentinal desensitizing agents such as potassium nitrate, strontium chloride or sodium fluoride; fluorides such as sodium fluoride, stannour fluoride, MFP; anesthetic agents such as lidocaine or benzocaine; anti-fungals such as those for the treatment of *candida albicans*; aromatics such as camphor, eucalyptus oil, and aldehyde derivatives such as benzaldehyde; insulin; steroids; herbal and other plant derived remedies; baking soda and anti-neoplastics. It is recognized that in certain forms of therapy, combinations of these agents in the same delivery system may be useful in order to obtain an optimal effect. Thus, for example, an antimicrobial and an anti-inflammatory agent may be combined in a single delivery system to provide combined effectiveness.

### Process for Preparation of the Composition

A process for preparing denture adhesive compositions of the present invention (creams, powders, wafers, non-aqueous liquids, aerosols, pastes) comprises conventional methods disclosed in the art. Conventional methods are taught in US 5,525,652, issued June 11, 1996, Clarke et al.; US 3,003,988, issued Oct. 10, 1961, Germann et al.; US 5,073,604, Holeva et al., issued Dec. 17, 1991; and US 5,872,161, Liang et al., issued Feb. 16, 1999.

A process for the preparation of the present denture adhesive compositions comprising a non-adhesive self-supporting layer, comprises coating a weighed amount of the adhesive components onto the non-adhesive self-supporting layer. This process is disclosed in US 5,877,233, Liang et al, issued March 2, 1999; US 5,872,160, issued 2/16/99, Liang et al.; US 5,880,172, Rajaiah et al., filed Oct. 25, 1996.

### Composition Use

The adhesive compositions may be in the form of a powder, cream, paste, non-aqueous liquid, aerosol, and/or wafer. Cream, paste, and other compositions of the present invention are generally applied to the denture prosthesis and thereafter the denture is secured to the oral cavity, as known in the art.

The following examples further describe and demonstrate embodiments within the scope of the present invention.

### EXAMPLE I

Denture stabilizing compositions in cream form are made by blending together the following ingredients:

| | A | B | C | D | E |
|---|---|---|---|---|---|
| | Grams | Grams | Grams | Grams | Grams |
| White Mineral Oil | 23.93 | 23.93 | 23.93 | 23.93 | 23.93 |
| Petrolatum, White | 19.8 | 8.87 | 14.37 | 16.87 | 16.87 |
| Carboxymethylcellulose Sodium | 20.00 | 20.00 | 20.00 | 20.00 | 20.00 |
| Silicon Dioxide, Colloidal | 1.14 | 1.14 | 1.14 | 1.14 | 1.14 |
| Colorant (Opatint Red Dye) | 0.06 | 0.06 | 0.06 | 0.06 | 0.06 |
| A salt of AVE/MA and/or AVE/MA/IB | 33.00 | 33.00 | 33.00 | 33.00 | 33.00 |
| Tetra Sodium Pyro Phosphate (TSPP) | 2.05 | - | 2.50 | | |
| EHDP (ethanehydroxy-1,1,-diphosphonic acid) | | | | 5.00 | |
| AHP (azacycloheptane-2,2-diphosphonic acid) | | | | | 5.00 |
| Poly Phosphate (Glass-H¹) | - | 13.00 | 5.00 | | |

| | | | | | |
|---|---|---|---|---|---|
| ¹ Polyphosphate manufactured by FMC Corporation where n=21 (average). | | | | | |

The red dye, petrolatum, and mineral oil are weighed, heated and mixed in a glass jar at 50 to 60 °C until visually uniform. The powders (colloidal silicon dioxide, CMC, salt of AVE/MA, salt of AVE/MA/IB and TSPP or AHP or EHDP or Glass-H) are then weighed and shake blended together in a container. Thereafter, the powders are mixed into the liquid with a spatula until visually a uniform pink cream. The above compositions can be modified by increasing or decreasing the level of TSPP by 0 to 15 grams, Glass-H by 0 to 15 grams, AVE/MA salt or AVE/MA/IB salt by 0 to 15 grams, petrolatum by 0 to 15 grams, and/or the CMC by 0 to 15 grams. The subject places from 0.1 to 5 grams of the cream composition on the denture. Then the subject inserts the denture into his/her mouth and presses it into place.

### EXAMPLE II

Denture stabilizing compositions in powder form are made by blending together the following ingredients:

| | A | B | C | D | E |
|---|---|---|---|---|---|
| | | | | | |
| Carboxymethylcellulose Sodium | 20.00 | 20.00 | 20.00 | 20.00 | 20.00 |
| Silicon Dioxide, Colloidal | 1.14 | 1.14 | 1.14 | 1.14 | 1.14 |
| A salt of AVE/MA and/or AVE/MA/IB | 33.00 | 33.00 | 33.00 | 33.00 | 33.00 |
| Tetra Sodium Pyro Phosphate (TSPP) | 2.05 | - | 2.50 | | |
| EHDP (ethanehydroxy-1,1,-diphosphonic acid) | | | | 5.00 | |
| AHP (azacycloheptane-2,2-diphosphonic acid) | | | | | 5.00 |
| Poly Phosphate (Glass-H¹) | - | 13.00 | 5.00 | | |

All components are blended together. The above compositions can be modified by increasing or decreasing the level of TSPP by 0 to 15 grams, Glass-H by 0 to 15 grams, AHP by 0 to 15 grams, EHDP by 0 to 15 grams, salt of AVE/MA or salt of AVE/MA/IB by 0 to 15 grams, and/or the CMC by 0 to 15 grams. The subject places from 0.1 to 5 grams of the composition on a denture and then moistens it. Then the subject inserts the denture into his/her mouth and presses it into place.

### EXAMPLE III

Denture stabilizing compositions in wafer form can be made by wetting a 58" by 20" non-woven polyester (non-adhesive self-supporting layer) with water. This wet sheet is uniformly coated with 1 to 3 times the weight of compositions listed above in example-II. Thereafter, the layer is rewetted with water. The layer is dried. The composition is mechanically softened by ring-roller, and then the composition is smoothed on a hydraulic press. The composition is die cut into desired shapes. These wafer compositions are moistened and applied to the dentures. Then the denture is inserted into the mouth and pressed into place.

## Claims

1. A denture adhesive composition comprising:
a) from 20 to 50%, by weight of the composition, of a denture adhesive component selected from salts of copolymers of an alkyl vinyl ether and maleic acid or maleic anhydride; salts of terpolymers of an alkyl vinyl ether, maleic acid or maleic anhydride and isobutylene; and mixtures thereof;
b) from 4.5 to 40%, by weight of the composition, of an anticalculus agent selected from salts of linear polyphosphates, having a chain length of two or more phosphate groups, and salts of diphosphonates; and
c) a non-aqueous denture adhesive carrier.

2. The composition of Claim 1 wherein the denture adhesive component is a salt of a copolymer of an alkyl vinyl ether and maleic acid or maleic anhydride, the salt containing a cationic salt function comprising a cation selected from strontium, zinc, iron, magnesium, calcium, sodium and mixtures thereof.

3. The composition of Claim 1 wherein the denture adhesive component is a mixed salt of a copolymer of an alkyl vinyl ether and maleic acid or maleic anhydride containing a cationic salt function comprising two or more cations selected from strontium, zinc, iron, magnesium, calcium, sodium and mixtures thereof.

4. The composition of Claim 1 wherein the denture adhesive component is a salt of a copolymer of an alkyl vinyl ether and maleic acid or maleic anhydride with a free acid level of from 36% to 60%.

5. The composition of Claim 1 wherein the anticalculus agent is selected from alkali metal pyrophosphates, di-, tri-, and mono-potassium or sodium pyrophosphates, dialkali metal pyrophosphate salts, tetraalkali metal pyrophosphate salts, and mixtures thereof; preferably trisodium pyrophosphate, disodium dihydrogen pyrophosphate (Na₂H₂P₂O₇), dipotassium pyrophosphate, tetrasodium pyrophosphate (Na₄P₂O₇), tetrapotassium pyrophosphate (K₄P₂O₇), and mixtures thereof

6. The composition of Claim 1 wherein the anticalculus agent is a linear, "glassy" polyphosphate salt having the formula: XO(XPO₃)ₙX where X is sodium or potassium and n averages from 6 to 125.

7. The composition of Claim 1 wherein the non-aqueous denture adhesive carrier is selected from a non-aqueous vehicle and a non-adhesive self supporting layer.

8. The composition of Claim 7 wherein the non-aqueous vehicle is selected from liquid petrolatum, petrolatum, mineral oil, glycerin, natural and synthetic oils, fats, silicone and silicone derivatives, polyvinylacetate, natural and synthetic waxes, vegetable oil waxes, vegetable oils, and mixtures thereof; preferably liquid petrolatum, petrolatum, mineral oil, glycerin, and mixtures thereof.

9. The composition of Claim 7 wherein the carrier is a non-adhesive self-supporting layer, selected from polyester, polypropylene, nylon, rayon, cellulose acetate, non-adhesive cellulose derivatives, cloth, fibrous fleece, paper, plastic, leather, synthetic fibers, natural fibers, and mixtures, thereof.

10. The use of an anticalculus agent selected from linear polyphosphate salts having a chain length of two or more phosphate groups, and diphosphonate salts in the manufacture of a denture adhesive composition according to any preceding claim for reducing or preventing calculus or tartar on teeth in the oral cavity, by application of the denture adhesive composition to a denture prosthesis or to the oral cavity and thereafter securing the denture prosthesis to the oral cavity.

## Patentansprüche

1. Gebisshaftmittelzusammensetzung, umfassend:
a) 20 bis 50 Gew.-% der Zusammensetzung einer Gebisshaftmittelkomponente, gewählt aus Salzen von Copolymeren aus einem Alkylvinylether und Maleinsäure oder Maleinsäureanhydrid; Salzen von Terpolymeren aus einem Alkylvinylether, Maleinsäure oder Maleinsäureanhydrid und Isobutylen; und Mischungen hiervon;
b) 4,5 bis 40 Gew.-% der Zusammensetzung eines Zahnsteinbildungsverhinderungsmittels, gewählt aus Salzen linearer Polyphosphate mit einer Kettenlänge von zwei oder mehreren Phosphatgruppen und Salzen von Diphosphonaten; und
c) einen nichtwässrigen Gebissklebemittelträger.

2. Zusammensetzung nach Anspruch 1, wobei die Gebissklebemittelkomponente ein Salz eines Copolymeren aus einem Alkylvinylether und Maleinsäure oder Maleinsäureanhydrid ist, wobei das Salz eine kationische Salzfünktion enthält, umfassend ein Kation, gewählt aus Strontium, Zink, Eisen, Magnesium, Calcium, Natrium und Mischungen hiervon.

3. Zusammensetzung nach Anspruch 1, wobei die Gebissklebemittelkomponente ein gemischtes Salz eines Copolymeren aus einem Alkylvinylether und Maleinsäure oder Maleinsäureanhydrid ist, enthaltend eine kationische Salzfunktion, umfassend zwei oder mehrere Kationen, gewählt aus Strontium, Zink, Eisen, Magnesium, Calcium, Natrium und Mischungen hiervon.

4. Zusammensetzung nach Anspruch 1, wobei die Gebissklebemittelkomponente ein Salz eines Copolymeren aus einem Alkylvinylether und Maleinsäure oder Maleinsäureanhydrid mit einem Gehalt an freier Säure von 36% bis 60% ist.

5. Zusammensetzung nach Anspruch 1, wobei das Zahnsteinbildungsverhinderungsmittel gewählt ist aus Alkalimetallpyrophosphaten, Di-, Tri- und Monokalium- oder -natriumpyrophosphaten. Dialkalimetallpyrophosphatsalzen, Tetraalkalimetallpyrophosphatsalzen und Mischungen hiervon; vorzugsweise Trinatriumpyrophosphat, Dinatriumdihydrogenpyrophosphat (Na₂H₂P₂O₇), Dikaliumpyrophosphat, Tetranatriumpyrophosphat (Na₄P₂O₇), Tetrakaliumpyrophosphat (K₄P₂O₇) und Mischungen hiervon.

6. Zusammensetzung nach Anspruch 1, wobei das Zahnsteinbildungsverhinderungsmittel ein lineares "glasartiges" Polyphosphatsalz der Formel XO(XPO₃)ₙX ist, worin X Natrium oder Kalium ist und n durchschnittlich 6 bis 125 beträgt.

7. Zusammensetzung nach Anspruch 1, wobei der nichtwässrige Gebissklebemittelträger aus einem nichtwässrigen Träger und einer nichthaftenden selbsttragenden Schicht gewählt ist.

8. Zusammensetzung nach Anspruch 7, wobei der nichtwässrige Träger gewählt ist aus flüssigem Petrolatum, Petrolatum, Mineralöl, Glycerin, natürlichen und synthetischen Ölen, Fetten, Silicon und Siliconderivaten, Polyvinylacetat, natürlichen und synthetischen Wachsen, pflanzlichen Ölwachsen, pflanzlichen Ölen und Mischungen hiervon; vorzugsweise flüssiges Petrolatum, Petrolatum, Mineralöl, Glycerin und Mischungen hiervon.

9. Zusammensetzung nach Anspruch 7, wobei der Träger eine nichthaftende selbsttragende Schicht ist, gewählt aus Polyester, Polypropylen, Nylon, Rayon, Celluloseacetat, nichthaftenden Cellulosederivaten, Stoff, faseriges Vlies, Papier, Kunststoff, Leder, synthetischen Fasern, natürlichen Fasern und Mischungen hiervon.

10. Verwendung eines Zahnsteinbildungsverhinderungsmittels, gewählt aus linearen Polyphosphatsalzen mit einer Kettenlänge von zwei oder mehreren Phosphatgruppen und Diphosphonatsalzen zur Herstellung einer Gebissklebemittelzusammensetzung gemäß mindestens einem vorangehenden Anspruch zur Verringerung oder Verhinderung von Zahnstein oder Belag auf Zähnen in der Mundhöhle durch Aufbringen der Gebissklebemittelzusammensetzung auf eine Zahnprothese oder auf die Mundhöhle und danach Befestigen der Zahnprothese in der Mundhöhle.

## Revendications

1. Composition adhésive pour prothèse dentaire comprenant :
a) 20 à 50 %, en poids de la composition, d'un composant adhésif pour prothèse dentaire choisi parmi des sels de copolymères d'un éther alkylvinylique et d'acide maléique ou d'anhydride maléique ; des sels de terpolymères d'un éther alkylvinylique, d'acide maléique ou d'anhydride maléique et d'isobutylène ; et leurs mélanges ;
b) 4,5 à 40 % en poids de la composition, d'un agent antitartre choisi parmi des sels de polyphosphates linéaires, ayant une longueur de chaîne de deux groupes phosphates ou plus, et des sels de diphosphonates ; et
c) un véhicule non aqueux d'adhésif pour prothèse dentaire.

2. Composition selon la revendication 1, dans laquelle le composant adhésif pour prothèse dentaire est un sel d'un copolymère d'un éther alkylvinylique et d'acide maléique ou d'anhydride maléique, le sel contenant une fonction de sel cationique comprenant un cation choisi parmi le strontium, le zinc, le fer, le magnésium, le calcium, le sodium et leurs mélanges.

3. Composition selon la revendication 1, dans laquelle le composant adhésif pour prothèse dentaire est un sel mixte d'un copolymère d'un éther alkylvinylique et d'acide maléique ou d'anhydride maléique contenant une fonction de sel cationique comprenant deux cations ou plus choisis parmi le strontium, le zinc, le fer, le magnésium, le calcium, le sodium et leurs mélanges.

4. Composition selon la revendication 1, dans laquelle le composant adhésif pour prothèse dentaire est un sel d'un copolymère d'un éther alkylvinylique et d'acide maléique ou d'anhydride maléique avec une quantité d'acide libre de 36 % à 60 %.

5. Composition selon la revendication 1, dans laquelle l'agent antitartre est choisi parmi des pyrophosphates de métaux alcalins, des pyrophosphates de di-, tri-, et mono-potassium ou sodium, des sels de pyrophosphates de dimétaux alcalins, des sels de pyrophosphates de tétramétaux alcalins, et leurs mélanges ; de préférence le pyrophosphate trisodique, le pyrophosphate disodique dihydrogéné (Na₂H₂P₂O₇), le pyrophosphate dipotassique, le pyrophosphate tétrasodique (Na₄P₂O₇), le pyrophosphate tétrapotassique (K₄P₂O₇), et leurs mélanges.

6. Composition selon la revendication 1, dans laquelle l'agent antitartre est un sel de polyphosphate linéaire, "vitreux" ayant la formule XO(XPO₃)ₙX, dans laquelle X est le sodium ou le potassium et n est une moyenne de 6 à 125.

7. Composition selon la revendication 1, dans laquelle le véhicule non aqueux d'adhésif pour prothèse dentaire est choisi parmi un véhicule non aqueux et une couche auto-supportée non adhésive.

8. Composition selon la revendication 7, dans laquelle le véhicule non aqueux est choisi parmi le pétrolatum liquide, le pétrolatum, l'huile minérale, la glycérine, des huiles naturelles et synthétiques, des graisses, des silicones et des dérivés de silicone, le polyacétate de vinyle, des cires naturelles et synthétiques, des cires d'huiles végétales, des huiles végétales, et leurs mélanges ; de préférence le pétrolatum liquide, le pétrolatum, l'huile minérale, la glycérine, et leurs mélanges.

9. Composition selon la revendication 7, dans laquelle le véhicule est une couche auto-supportée non adhésive, choisie parmi le polyester, le polypropylène, le nylon, la rayonne, l'acétate de cellulose, des dérivés cellulosiques non adhésifs, des étoffes, des toisons fibreuses, du papier, du plastique, du cuir, des fibres synthétiques, des fibres naturelles, et leurs mélanges.

10. Utilisation d'un agent antitartre choisi parmi des sels de polyphosphates linaires ayant une longueur de chaîne de deux groupes phosphates ou plus, et des sels de diphosphonates, dans la fabrication d'une composition adhésive pour prothèse dentaire selon l'une quelconque des revendications précédentes pour réduire ou éviter la formation de tartre sur les dents dans la cavité orale, par application de la composition adhésive pour prothèse dentaire sur une prothèse dentaire ou dans la cavité orale, puis par fixation de la prothèse dentaire dans la cavité orale.
